# EUROPÄISCHE PATENTSCHRIFT

(11) **EP 1 059 359 B1**
(45) Veröffentlichungstag und Bekanntmachung des Hinweises auf die Patenterteilung: **04.01.2006**
(21) Anmeldenummer: 00111738.1
(22) Anmeldetag: 02.06.2000
(51) Int. Cl.: C12Q 1/37, G01N 33/573

(54) **Verfahren zur Bestimmung der Aktivität der Faktor VII-aktivierenden Protease aus Proteinlösungen**
Method for the determination of the activity of the Factor VII-activating protease in protein solutions
Méthode pour la determination de l'activité de la protéase qui active le Factor VII dans des solutions protéiques

(30) Priorität: 10.06.1999 DE 19926531; 17.05.2000 DE 10023923
(43) Veröffentlichungstag der Anmeldung: 13.12.2000
(60) Teilanmeldung: 05023845.0
(73) Patentinhaber: ZLB Behring GmbH, 35041 Marburg (DE)
(72) Erfinder: Römisch, Jürgen, Dr., 35041 Marburg (DE); Feussner, Annette, 35043 Marburg (DE); Stöhr, Hans-Arnold, 35093 Wetter (DE)

(56) Entgegenhaltungen:
- EP-A- 0 769 559
- EP-A- 0 952 215
- ROEMISCH J ET AL: "A PROTEASE ISOLATED FROM HUMAN PLASMA ACTIVATING FACTOR VII INDEPENDENT OF TISSUE FACTOR" BLOOD COAGULATION & FIBRINOLYSIS, RAPID COMMUNICATIONS, OXFORD,OXFORD, GB, Bd. 10, Nr. 8, Dezember 1999 (1999-12), Seiten 471-479, XP001036685 ISSN: 0957-5235
- ZHUKOV A ET AL: "Purification and characterization of hepsin from rat liver microsomes" BIOCHIMICA ET BIOPHYSICA ACTA. PROTEIN STRUCTURE AND MOLECULAR ENZYMOLOGY, ELSEVIER, AMSTERDAM,, NL, Bd. 1337, Nr. 1, 4. Januar 1997 (1997-01-04), Seiten 85-95, XP004281512 ISSN: 0167-4838
- ROEMISCH J ET AL: "THE FVII ACTIVATING PROTEASE MEDIATES FIBRINOLYTIC EFFECTS ACTIVATING SINGLE-CHAIN PLASMINOGEN ACTIVATORS" ANNALS OF HEMATOLOGY, BERLIN, DE, Bd. 78, Nr. SUPPL 1, 24. Februar 1999 (1999-02-24), Seite A24 XP001059495 ISSN: 0939-5555
- ROEMISCH J ET AL: "A PROTEASE ISOLATED FROM PLASMA WHICH ACTIVATES FVII IN A TISSUE FACTOR INDEPENDENT MANNER BU INACTIVATES FV AND FVIII" ANNALS OF HEMATOLOGY, BERLIN, DE, Bd. 78, Nr. SUPPL 1, 24. Februar 1999 (1999-02-24), Seite A10 XP001059496 ISSN: 0939-5555
- HUNFELD A ET AL: "IDENTIFICATION OF THE THROMBIN-LIKE ACTIVITY OF PCCS" ANNALS OF HEMATOLOGY, BERLIN, DE, Bd. 76, Nr. SUPPL 1, 25. Februar 1998 (1998-02-25), Seite A101 XP008000532 ISSN: 0939-5555
- ETSCHEID M ET AL: "CHARACTERISATION OF A NOVEL SERINE PROTEASE FROM PLASMA" ANNALS OF HEMATOLOGY, BERLIN, DE, Bd. 78, Nr. SUPPL 1, 24. Februar 1999 (1999-02-24), Seite A42 XP001059494 ISSN: 0939-5555
- CHOI-MIURA N H ET AL: "PURIFICATION AND CHARACTERIZATION OF A NOVEL HYALURONAN-BINDING PROTEIN (PHBP) FROM HUMAN PLASMA: IT HAS THREE EGF, A KRINGLE AND ASERINE PROTEASE DOMAIN, SIMILAR TO HEPATOCYTE GROWTH FACTOR ACTIVATOR" JOURNAL OF BIOCHEMISTRY, JAPANESE BIOCHEMICAL SOCIETY, TOKYO, JP, Bd. 119, Nr. 6, 1996, Seiten 1157-1165, XP000960750 ISSN: 0021-924X
- ROEMISCH JUERGEN ET AL: "The FVII activating protease cleaves single-chain plasminogen activators." HAEMOSTASIS, Bd. 29, Nr. 5, Oktober 1999 (1999-10), Seiten 292-299, XP001053403 ISSN: 0301-0147
- HUNFELD A ET AL: "Detection of a novel plasma serine protease during purification of vitamin K-dependent coagulation factors" FEBS LETTERS, ELSEVIER SCIENCE PUBLISHERS, AMSTERDAM, NL, Bd. 456, Nr. 2, 6. August 1999 (1999-08-06), Seiten 290-294, XP004260083 ISSN: 0014-5793

## Beschreibung

Gegenstand der Erfindung ist ein Verfahren zur qualitativen und quantitativen Bestimmung der Faktor VII-aktivierenden Protease in komplexen Proteinlösungen wie Plasma.

Aus der deutschen Patentanmeldung 199 03 693.4 sind bereits Testsysteme und Verfahren zum qualitativen und quantitativen Nachweis einer Protease bekannt, die den Blutgerinnungsfaktor VII aktiviert. Diese umfassen chromogene Testverfahren, die auf der Spaltung markierter, niedermolekularer Peptidsubstrate und der photometrischen Bestimmung der dabei auftretenden Extinktion beruhen, sowie Testverfahren, bei denen die biologischen Eigenschaften der genannten Protease ausgenutzt werden. Dabei kann die Protease oder ihr Proenzym dadurch nachgewiesen werden, dass sie
a) eine die Blutgerinnungsfaktoren VIII/VIIIa oder VNa inaktivierende Wirkung oder
b) in globalen Gerinnungstests eine die Blutgerinnungszeiten verkürzende Wirkung oder
c) eine Plasminogen-Aktivatoren aktivierende Wirkung oder
d) eine den FVII aktivierende Wirkung aufweist.

Die Bestimmung der Aktivität des FVII-Aktivators führt bei den bisher eingesetzten Bestimmungsverfahren jedoch nur dann zu zuverlässigen Ergebnissen, wenn die Protease in gereinigtem oder angereichertem Zustand vorliegt und keine störenden Einflüsse von Verunreinigungen das Messergebnis verfälschen. Besonders sehr komplexe Proteingemische wie Plasma oder Gewebeflüssigkeiten enthalten nun allerdings eine Vielzahl von Proteinen, die eine spezifische Bestimmung des Faktor VII-Aktivators verhindern oder zumindest behindern können. Außerdem liegt die Protease nach dem derzeitigen Wissensstand im Plasma vor allem als Proenzym vor, so dass eine Aktivierung zur aktiven Protease zum Zwecke der anschließenden Aktivitätsbestimmung nötig ist.

Die Faktor VII-aktivierende Protease ist ein Plasmaprotein, das an der Regulation der Hämostase, vor allem durch Mitwirkung an Gerinnungs- und Fibrinolyseprozessen, beteiligt ist. Deshalb ist die Untersuchung der Funktion und der biologischen Aktivität der Protease von hohem Interesse. So könnte zum Beispiel ein emiedrigter Antigengehalt und/oder eine Störung der biologischen Aktivität, beispielsweise durch eine Genmutation, ein erhöhtes Thromboserisiko anzeigen. Es stellte sich deshalb die Aufgabe, ein Verfahren zu entwickeln, das die möglichst einfache und spezifische Bestimmung einer oder mehrerer biologischer Aktivitäten der Faktor VII-aktivierenden Protease ermöglicht Da die physiologische Aufgabe dieser Protease bisher noch unklar ist, sollte diese Methode prinzipiell die Bestimmung mehrerer Aktivitäten erlauben.

Es wurde nun gefunden, dass diese Anforderungen durch ein Verfahren zur Bestimmung der Aktivität der den Blutgerinnungsfaktor VII aktivierenden Protease in Proteinlösungen erfüllt werden, bei dem
- die der Protease und/oder ihr Proenzym enthaltende Proteinlösung mit einer Festphase inkubiert wird, an die zuvor ein gegen die Protease gerichteter Antikörper gekoppelt wurde und
- nach Auswaschen der Festphase die daran fixierte Protease und/oder ihr Proenyzm mit Reagenzien inkubiert werden, die deren Aktivitätsbestimmung erlauben.

Dieses Bestimmungsverfahren lässt sich überraschenderweise nicht nur auf die Protease in ihrer aktivierten Form anwenden. Obwohl die bisherigen Untersuchungsergebnisse darauf schließen lassen, dass die Protease im Plasma hauptsächlich als Proenzym zirkuliert und es daher zu erwarten war, dass dieses nach Bindung an die oben genannte Festphase erst noch aktiviert werden muss, um danach ihre biologischen Aktivitäten entfalten zu können, wurde nun überraschenderweise gefunden, dass eine solche Aktivierung nicht notwendig ist, sondern dass das aus dem Plasma an die Festphase gebundene Proenzym in immobilisierter Form seine biologische Aktivität in gleicher Weise entfaltet wie das aktive Enzym. Dadurch erübrigt sich ein gesonderter Aktivierungsschritt und ermöglicht eine weitaus schnellere und störungsfreiere Bestimmung.

Eine spezifische Aktivierung des Proenzyms kann aber durchaus noch angeschlossen werden, um sicherzustellen, dass das gebundene Proenzym vollständig aktiviert wurde.

Als Festphase kommen die dem Fachmann bekannten Matrices wie aktivierte Sepharose® oder Fraktogel® in Frage. Vorzugsweise werden Mikrotiterplatten mit gegen die Protease gerichteten Antikörpern beschichtet, die polyklonaler oder monoklonaler Herkunft sein können. Auch Antikörperfragmente wie F(ab) oder F(ab)₂ können verwendet werden. Im Gegensatz zum Antigentest, in dem ein markierter Zweitantikörper zur Detektion und dann auch zur Quantifizierung verwendet wird, werden erfindungsgemäß chromogene Substrate zugesetzt, die die Aktivitätsbestimmung der Protease zulassen. Ein besonders bevorzugtes chromogenes Substrat ist S2288 von Chromogenix AB (H-D-isoleucyl-L-prolyl-L-arginin-pNA x 2 HCl), das ebenso wie ähnliche Verbindungen eine signifikante konzentrations- und zeitabhängige Zunahme der Absorption durch Amidolyse des Substrats zeigt. Überraschenderweise behält die Protease ihre biologischen Aktivitäten und Eigenschaften auch nach Bindung an den Antikörper bei, nämlich die Fähigkeit FVII und Plasminogen-Aktivatoren zu aktivieren. Dadurch wird die spezifische Bestimmung der Funktionalität der Protease aus einer komplexen Proteinlösung heraus möglich.

Neben den chromogenen Substraten bieten sich auch die übrigen in der deutschen Patentanmeldung 199 03 693.4 erwähnten Substrate zur Aktivitätsbestimmung an, also die Inaktivierung der Blutgerinnungsfaktoren VIII/VIIIa oder V/Va und auch die Aktivierung des FVII und der Plasminogenaktivatoren. Dabei kann zum Beispiel der so aktivierte Anteil an Faktor VII durch direkte Amidolyse eines für den FVII spezifischen chromogenen Substrats bestimmt werden oder durch eine gekoppelte Reaktion wie den sog. FVlla-rTF-Test. Die Aktivierung von Einkettenplasminogenaktivatoren scuPA (single chain urokinase plasminogen activator) oder der sctPA (single chain tissue plasminogen activator) lässt sich einfach durch Substratumsetzung von zum Beispiel S2444 (pyroGlu-Gly-Arg-pNA x HCl) verfolgen. Wie in der deutschen Patentanmeldung 199 03 693.4 beschrieben, können zum Nachweis auch Substanzen zugesetzt werden, die die Aktivität der Protease stimulieren, zum Beispiel lösliche Kalziumsalze und/oder Heparin oder dem Heparin verwandte Substanzen wie Dextransulfat.

Weitere Untersuchungen von Lösungen, Körperflüsigkeiten und Zell- bzw. Gewebeextrakten mit Hilfe des beschriebenen Verfahrens zeigten dessen Eignung zur Detektion bzw. Quantifizierung der FVII-aktivierenden Protease. Darunter sind die diese Protease enthaltende Lösungen zu verstehen, wie intermediate der Präparation der Protease, Zellkultur-Überstände, die auch bei der Fermentation entsprechender Zellen zur rekombinanten Expression anfallen. Darunter sind auch Lösungen zu verstehen, die bei der transgenen Herstellung der Protease bzw. des Proenzymes anfallen, wie Milch.

Darüber hinaus eignet sich das Verfahren zur Bestimmung der Aktivität der FVII-aktivierenden Protease in Extrakten von Geweben oder Zellen, die einen Eindruck über das Vorhandensein der Proteaseaktivität gibt bzw. über potentielle pathologische Zustände bei Über- oder Unterexpression dieses Proteins.

Ein besonderes Interesse gilt dem Nachweis der Proteaseaktivitäten in Körperflüssigkeiten, wie Blut und Plasma, Seminalplasma, Urin, Cerebrospinalflüssigkeit, Bronchioalveolar-Lavage, Fruchtwasser, Speichel oder Tränenflüssigkeit. Einen wertvolle Ergänzung zum Aktivitätstest stellt dabei das in der deutschen Patentanmeldung 199 03 693.4 erwähnte Antigenbestimmungssystem (z.B. ELISA) dar, da mit Hilfe beider Parameter ein umfassenderes Bild beispielsweise bei einer Erkrankung erhalten werden kann.

Bei der Untersuchung gesunder Blutspender fiel auf, daß ca. 5-10% der untersuchten Plasmen eine gegenüber einem Standard (Poolplasmen) deutlich geringere Proteaseaktivität aufwiesen (etwa 30-50% des 'Durchschnittswertes'), wie in Beispiel 1 näher ausgeführt. Diese Information wurde dadurch ergänzt, daß nahezu alle dieser Spender im Normalbereich befindliche Antigengehalte aufwiesen. Dies könnte beispielsweise auf (heterozygote) Mutation(en) hinweisen, die die Aktivität, jedoch nicht den Antigengehalt, einer Plasmaprobe entsprechend beeinflussen würden. Als Konsequenz könnten diese Donoren eine Risikogruppe für bestimmte Krankheiten darstellen und gegebenenfalls prophylaktische Maßnahmen frühzeitig ergriffen werden. Dies gilt sowohl für Personen, die erhöhte als auch emiedigte Aktivitätsspiegel aufweisen. Signifikant erhöhte Aktivitäten dieser Protease (bei teilweise normalem oder leicht erhöhten Antigengehalt) fanden wir in Plasmen von Patienten mit Herzinfarkten (siehe auch Beispiel 2) und stabiler und instabiler Angina pectoris verglichen mit einem Kollektiv gesunder Spender. Bislang ist noch nicht geklärt, ob eine Erhöhung der Proteaseaktivität als eine Ursache dieser Erkrankung gewertet werden kann oder ob diese eher einer Gegenreaktion des Körpers entspricht, im Sinne einer gesteigerten Thrombolyse.

Abgesehen von der physiologischen Relevanz der erhöhten Proteaseaktivitäten kann dieser Parameter zur frühen Erkennung und als Kriterium einer Veränderung des Krankheitsbildes verwendet werden. Dies schließt die Diagnostik weiterer Herz-Kreislauf assoziierter Komplikationen ein.

Über diese Indikationen hinaus kann das beschriebene Testsystem (auch in Kombination mit einer Antigenbestimmung) zur Diagnose und Therapieverfolgung auch bei malignen Erkrankungen, Entzündungen, Autoimmunerkrankungen, Vaskulitiden, respiratorischen Defekten bzw. zur Hämostasediagnostik (Gerinnung und Fibrinolyse), wie auch bei Sepsis und assoziierten Reaktionen, wie der disseminierten intravasalen Gerinnung verwendet werden. Weitere Anwendungsgebiete umschließen die Diagnose von Organdefekten, wie zerebralen, respiratorischen und Nierenerkrankungen. In Patienten mit Leberzirrhose fanden wir signifikant emiedrigte Aktivitäten der Protease, die in den meisten Fällen mit emiedrigten Antigenspiegeln einhergingen.

Weitere Untersuchungen zeigten, daß neben einem moderaten Anstieg des Antigenspiegels im Plasma gesunder Schwangerer ein deutlicher Anstieg der Proteaseaktivit im Verlauf von Schwangerschaften zu beobachten ist, mit höchsten Werten im dritten Trimester. Ein Ausbleiben eines solchen Anstieges der Protease kann mit einem Risiko für Mutter und Kind während der Schwangerschaft verbunden sein, wie thromboembolischen Komplikationen etc. bis hin zur Früh- und Fehlgeburt oder Mißbildung des Fötus.

Die Erfindung wird durch die folgenden Beispiele erläutert:

### Beispiel 1

Mikrotiterplatten (96 wells) wurden mit einem monoklonalen Antikörper gegen den FVII-Aktivator beschichtet, indem in jede Vertiefung 150 µl einer 10 µg/ml enthaltenden Lösung pipettiert wurde. Nach 16-stündiger Inkubation bei Raumtemperatur wurde die Platte mehrmals gewaschen. In die Vertiefungen wurden jeweils 100 µl steigender Konzentrationen gereinigter Protease bzw. verschiedene Verdünnungen eines Standardhumanplasmas (SHPL) pipettiert. Nach Inkubation bei 37°C wurden die Lösungen durch mehrmaliges Waschen entfernt und die Aktivitäten bestimmt.

In jede Vertiefung wurden 50 µl einer Prourokinase-Lösung (10µg/ml, American Diagnostica, US) pipettiert sowie 50 µl Puffer, der 30 mM CaCl₂ und 100 lU/ml Heparin enthielt. Zwei Minuten später wurden weitere 100 µl Puffer und 25 µl des Substrates S2444 (3 mM) hinzugegeben. Die Zunahme der Absorption bei 405 nm pro Minute wurde ermittelt.

| **Protease, gereinigt (µg/ml)** | **Δ mOD/min** | **SHPL (Verdünnung)** | Δ **mOD/min** |
|---|---|---|---|
| 0 | 0,4 | buffer | 0,4 |
| 0,1 | 7 | 1:200 | 0,4 |
| 0,2 | 12 | 1:100 | 0,9 |
| 0,4 | 18 | 1:50 | 7,5 |
| 0,6 | 22 | 1:33,3 | 8,4 |
| 0,8 | 24 | 1:25 | 15,2 |
| 1,0 | 27 | 1:20 | 24,8 |
| 2,0 | 34 | 1:10 | 31,2 |

### Beispiel 2

Die Beschichtung der Mikrotiterplatten und die Inkubation mit den Probelösungen wurde durchgeführt wie in Beispiel 1 beschrieben. Anstelle der Aktivierung der Prourokinase wurde die Aktivierung des Faktors VII bestimmt. Dazu wurden je 50µl Puffer, enthaltend 30 mM CaCl₂ und 100 IU/ml Heparin, für 2 Minuten bei Raumtemperatur in die Vertiefungen der Platte gegeben. Nach Zusatz von weiteren 100 µl Puffer und 25µl Spectrozym® Vlla (3 mM, American Diagnostica/US) wurde die Δ mOD/min bestimmt.

| **Protease, gereinigt (µg/ml)** | **Δ mOD/min** | **SHPL (Verdünnung)** | **Δ mOD/min** |
|---|---|---|---|
| 0 | 0,3 | buffer | 0,3 |
| 0,2 | 1,8 | 1:100 | 0,3 |
| 0,4 | 2,8 | 1:50 | 0,3 |
| 0,6 | 3,0 | 1:33,3 | 0,8 |
| 0,8 | 3,6 | 1:25 | 3,2 |
| 1,0 | 4,7 | 1:20 | 7,2 |
| 1,5 | 7,1 | 1:13,3 | 8,4 |
| 2,0 | 7,9 | 1:10 | 11,5 |

Mit Hilfe dieser Verdünnungsreihen ist es möglich, individuelle Plasmen zu vergleichen und die Funktionalität der Protease zu bestimmen. Durch Vergleich mit einem Standardhumanplasma, das einen Pool von hunderten von Einzelplasmen darstellt, können signifikante Abweichungen von der Norm erfasst werden. Die so ermittelte Aktivität sollte idealerweise in das Verhältnis zum Antigengehalt gesetzt werden, der zum Beispiel mittels ELISA bestimmt werden kann.

Ist die Proteasemenge bekannt, dann lässt sich die spezifische Aktivität der in der Proteinlösung enthaltenen Protease sowie dessen Proenzyms bestimmen.

### Beispiel 3

### Bestimmung der Proteaseaktivität in 190 Plasmen gesunder Spender

190 Citrat-Plasmen gesunder Personen, davon 140 Männer und 50 Frauen, wurden mit Hilfe des beschriebenen Aktivitätstestes untersucht. Um festzustellen, ob potentiell vom Durchschnittswert aller untersuchten Plasmen abweichende Aktivitäten mit einer entsprechenden Veränderung des Protease Antigenspiegels einhergingen, wurde ein ELISA verwendet, wie in der deutschen Patentanmeldung 199 03 693.4 beschrieben. Ein solcher ELISA zum Nachweis der Protease als Antigen ist mit Hilfe monoklonaler oder polyklonaler spezifischer Antikörper gegen diese Protease machbar.

**Abbildung (1)** zeigt die Proteaseaktivitäten der untersuchten gesunden Männer (A) und Frauen (B). Es wird deutlich, daß 5-10%, sowohl Männer als auch Frauen, eine gegenüber dem Durchschnitt deutlich verminderte Aktivität aufweisen.

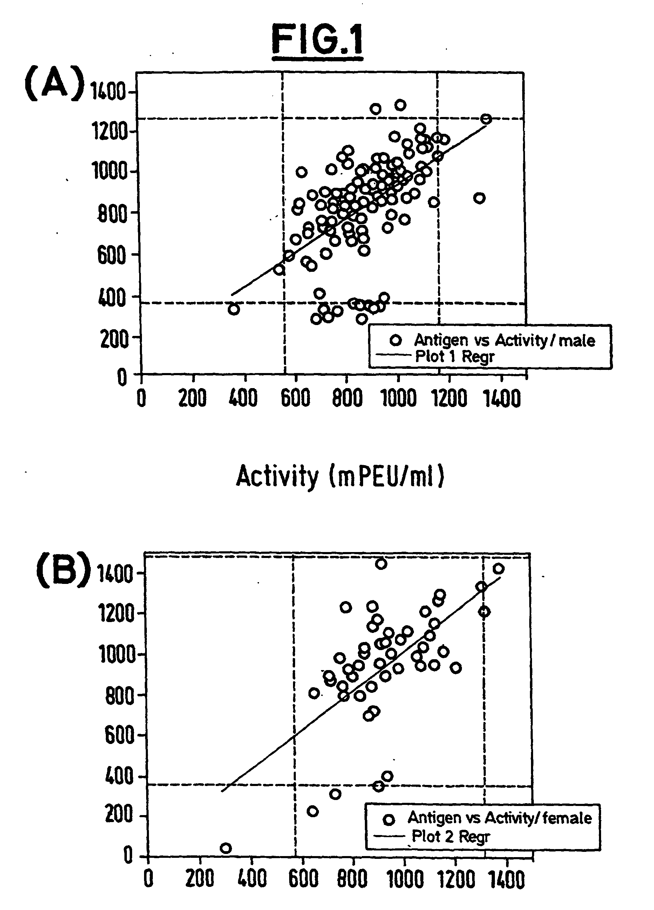

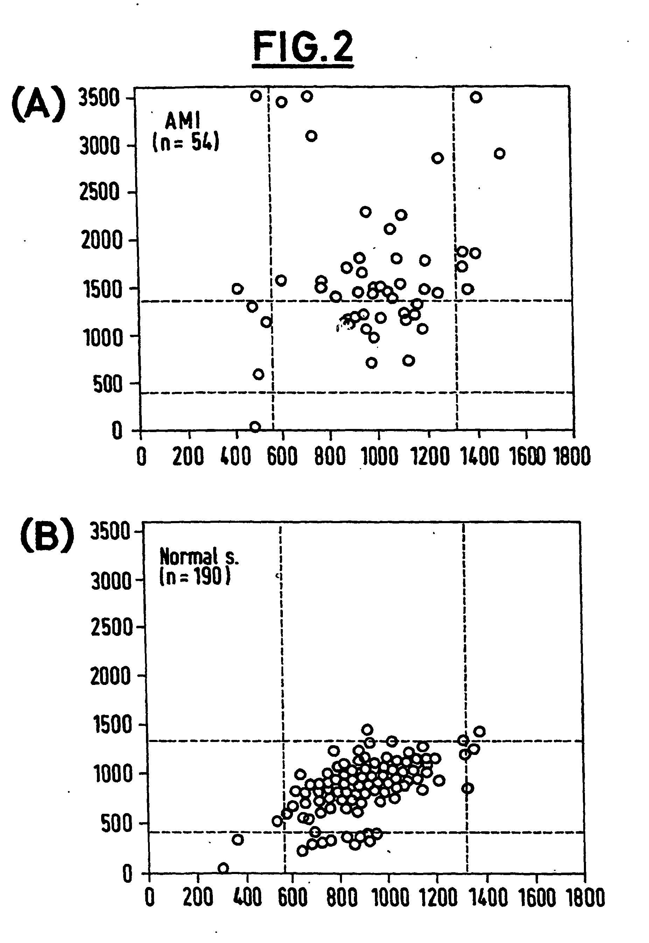

Die Proteaseaktivitäten (y-Achse) und die dazu gehörenden Antigenspiegel der entsprechenden Personen (x-Achse) sind in der Abbildung dargestellt. Die arbiträren 'Normalbereiche' der Antigen- und Aktivitätsspiegel sind durch waagerechte und senkrechte Linien als obere und untere Begrenzungen der Parameter jeweils gezeigt. Die sich daraus ergebenden Rechtecke (in jeweils der Mitte der Abbildung) stellen demnach die 'Normalbereiche' gesunder Spender dar. Hier wird nochmals besonders deutlich, daß die Mehrzahl der Proben mit verminderter Aktivität nicht mit einer entsprechenden Verminderung der Antigenspiegel einherging. Dies könnte auf eine heterozygote Mutation (oder mehrere) hinweisen, d.h. beispielsweise könnten ca. 50% der Proteasemoleküle durch eine oder mehrere Mutationen so verändert sein, daß eine Reaktion mit biologischen Substraten nicht mehr gewährleistet ist. Im Falle einer fibrinolytischen Bedeutung der Protease könnte dies mit einem Thromboserisiko (oder für andere Erkrankungen etc.) dieser derzeit noch 'gesunden' Population verbunden sein. Obwohl in der Minderzahl der untersuchten Proben, sind die Werte der reduzierten Proteaseaktivität, die sehr wohl mit einem verminderten Antigengehalt einhergehen, als nicht minder interessant zu bewerten, da offensichtlich eine Dysregulation der plasmatischen Verfügbarkeit der Protease vorliegt, die mit einem vergleichbaren Risiko wie beschrieben verbunden sein kann.

Entsprechend ist die Detektion der Proteaseaktivität, auch im Verbund mit der Antigenbestimmung, als ein Parameter zur Früherkennung und Prophylaxe-/Therapiekontrolle zu sehen.

### Beispiel 4

### Bestimmung der Proteaseaktivität in Plasmen schwangerer Frauen

Zitratplasmen schwangerer Frauen wurden, wie in Beispiel 3 beschrieben, getestet. Proben wurden an verschiedenen Zeitpunkten der Schwangerschaft gewonnen und anschließend untersucht.

Die Verläufe zweier unproblematischer Schwangerschaften sind in Tabelle 1 dargestellt. Ein deutlicher Anstieg der Proteaseaktivität mit Dauer der Schwangerschaft ist zu erkennen, wogegen die Antigegehalte der Protease nicht bis moderat ansteigen. Ein Ausbleiben dieser erhöhten Aktivität könnte mit Problemen für Mutter und Fötus verbunden sein.

Gesunde (nicht Schwangere) zeigen dagegen eine kontinuierlichen Verlauf der Proteaseaktivitäten (weder erhöht noch vermindert, im Rahmen der Testschwankungen) während einer entsprechend Beobachtungszeit (nicht dargestellt).

**Tabelle 1 Die prozentualen Angaben beziehen sich auf Durchschnittswerte gesunder (nicht schwangerer) Frauen.**

| Trimester der Schwangerschaft | Antigen (%) Schwangere | | Aktivität (%) Schwangere | |
|---|---|---|---|---|
| | 1 | 2 | 1 | 2 |
| I | 103 | 105 | 110 | 115 |
| II | 118 | 123 | 158 | 176 |
| III | 126 | 143 | 215 | 280 |

### Beispiel 5

### Bestimmung der Proteaseaktivität in Herzinfarktplasmen

Plasmen von 54 Patienten mit akutem Myokardinfarkt wurden bei Einlieferung (vor Intensivbehandlung) in die Notfallstation gewonnen und für die Routineanalytik verwendet. Später wurden Plasmareste (nicht aufgetaute Aliquots) zur Quantifizirung der Proteaseaktivitäten (und Antigengehalte) verwendet.

Die **AAbbildung (2)** faßt die Ergebnisse der Untersuchung zusammen. Gegenüber einem Kollektiv gesunder Spender (**B**) sind in Plasmen von Patienten mit akutem Myokardinfarkt (**A**) signifikant höhere Proteaseaktivitäten (und auch der Antigengehalte) zu messen.

Entsprechend können diese Parameter zur Früherkennung eines Infarktes, also auch bei stabiler und instabiler Angina pectoris, verwendet werden. Bei Patienten mit diesen koronaren Herzerkrankungen fanden wir ebenfalls im Durchschnitt signififkant erhöhte Aktivitäten. Die Höhe der gemessenen Werte kann eine Bewertung des Schweregrades der Krankheit ermöglichen bzw. im Verlaufe einer Infarkt- und Angina pectoris Prophylaxe und Therapie wertvolle Hinweise über den Zustand des Patienten geben. Darüber hinaus können diese Parameter zur Beurteilung anderer mit dem Herz-Kreislaufsystem asoziierter Komplikationen verwendet werden.

## Patentansprüche

1. Verfahren zur Bestimmung der Aktivität der den Blutgerinnungsfaktor VII aktivierenden Protease aus Proteinlösungen, **dadurch gekennzeichnet, dass**
- die die Protease und/oder ihr Proenzym enthaltende Proteinlösung mit einer Festphase inkubiert wird, an die zuvor ein gegen die Protease gerichteter Antikörper gekoppelt wurde und
- nach Auswaschen der Festphase die daran fixierte Protease und/oder ihr Proenzym mit Reagenzien inkubiert werden, die deren Aktivitätsbestimmung erlauben.

2. Verfahren nach Anspruch 1, **dadurch gekennzeichnet, dass** die Aktivität der Protease gemessen wird durch eine photometrische Bestimmung der bei Einwirkung auf chromogene Substrate auftretenden Extinktion.

3. Verfahren nach Anspruch 1, **dadurch gekennzeichnet, dass** die Aktivität der Protease gemessen wird durch
- ihre die Blutgerinnungsfaktoren VIII/VIIIa oder VNa inaktivierende Wirkung oder
- ihre die Blutgerinnungszeiten verkürzende Wirkung in globalen Gerinnungstests oder
- ihre Plasminogen-Aktivatoren aktivierende Wirkung oder
- ihre den FVII aktivierende Wirkung.

4. Verfahren nach den Ansprüchen 1 bis 3, **dadurch gekennzeichnet, daß** der gegen die Protease gerichtete Antikörper ein polyklonaler oder monoklonaler Antikörper oder ein F(ab) oder F(ab)₂-Antikörperfragment ist.

## Claims

1. A procedure for the determination of the activity of the protease which activates blood clotting factor VII from protein solutions, **wherein**
- the protein solution comprising the protease and/or its proenzyme is incubated with a solid phase to which an antibody directed against the protease has been coupled beforehand and
- after washing the solid phase the protease and/or its proenzyme fixed thereto are incubated with reagents which allow determination of their activity.

2. The procedure as claimed in claim 1, **wherein** the activity of the protease is measured by means of a photometric determination of the extinction occurring during action on chromogenic substrates.

3. The procedure as claimed in claim 1, **wherein** the activity of the protease is measured by means of
- its action which inactivates blood clotting factors VIII/VIIIa or V/Va or
- its action which shortens the blood clotting times in global clotting tests or
- its action which activates plasminogen activators or
- its action which activates FVII.

4. The procedure as claimed in claims 1 to 3, **wherein** the antibody directed against the protease is a polyclonal or monoclonal antibody or an F(ab) or F(ab)₂ antibody fragment.

## Revendications

1. Procédé de détermination de l'activité de la protéase d'activation du facteur de coagulation VII dans des solutions de protéines, **caractérisé en ce que**
- la solution de protéines qui contient la protéase et/ou son proenzyme est incubée avec une phase solide, à laquelle a été couplé préalablement un anticorps dirigé contre la protéase et
- après lavage de la phase solide la protéase qui y est fixée et/ou son proenzyme sont incubés avec des réactifs qui permettent la détermination de leur activité.

2. Procédé selon la revendication 1, **caractérisé en ce que** l'activité de la protéase est mesurée par une détermination photométrique de l'extinction provoquée par l'action sur un substrat chromogène.

3. Procédé selon la revendication 1, **caractérisé en ce que** l'activité de la protéase est mesurée par
- son action d'inactivation des facteurs de coagulation VIII/VIIIa V/Va ou
- son action de réduction du temps de coagulation dans des tests de coagulation globale ou
- son action d'activation des activateurs du plasminogène ou
- son action d'activation du FVII.

4. Procédé selon les revendications 1 à 3, **caractérisé en ce que**, l'anticorps dirigé contre la protéase est un anticorps polyclonal ou monoclonal ou un fragment d'anticorps F(ab) ou F(ab)₂.
